# EUROPEAN PATENT SPECIFICATION

(11) **EP 2 049 903 B1**
(45) Date of publication and mention of the grant of the patent: **09.09.2015**
(21) Application number: 07804639.8
(22) Date of filing: 25.07.2007
(51) Int. Cl.: G01N 33/558

(54) **AN IMMUNOCHROMATOGRAPHY DEVICE FOR THE DIAGNOSIS OF DISEASES IN A SAMPLE**
IMMUNCHROMATOGRAPHISCHE VORRICHTUNG ZUR DIAGNOSE VON KRANKHEITEN IN EINER PROBE
DISPOSITIF D'IMMUNOCHROMATOGRAPHIE UTILISÉ POUR LE DIAGNOSTIC DE MALADIES A PARTIR D'UN ÉCHANTILLON

(30) Priority: 25.07.2006 WO PCT/IB2006/002021
(43) Date of publication of application: 22.04.2009
(60) Divisional application: 12150192.8
(73) Proprietor: Augurix S.A., 1870 Monthey (CH)
(72) Inventor: BESSON DUVANEL, Cécile, 1815 Clarens (CH); DUVANEL, Thierry, 1815 Clarens (CH)
(74) Representative: KATZAROV S.A.
(86) International application number: PCT/IB2007/002112
(87) International publication number: WO 2008/012650

(56) References cited:
- EP-A- 1 164 375
- EP-A- 1 197 754
- EP-A- 1 329 724
- WO-A-95/04280
- WO-A-2004/016065
- WO-A-2005/082254
- WO-A-2007/024633
- US-A- 5 268 148
- US-A1- 2002 098 512
- US-B1- 6 203 757

## Description

### FIELD OF THE INVENTION

The present invention relates to the biotechnological field, particularly to biomedical diagnosis. More precisely, the invention concerns an immunochromatography device and a method for determining the presence of a binding analyte in a biological sample of a subject.

### BACKGROUND OF THE INVENTION

Celiac disease (CD), which is considered as an auto-immune disease, is one of the most common T-cell immune-mediated disorders and. CD is triggered in genetically susceptible individuals when ingesting toxic components (gluten) of specific cereals, wheat, rye and barley. Dietary gluten peptides cross the intestinal epithelium and initiate a series of cascades promoting inflammatory mechanisms and clonal expansion of B cells producing specific antibodies against gluten peptides (gliadin) and an autoimmune response against tissue transglutaminase (Tgase2), an ubiquitous intracellular enzyme responsible for gluten deamidation [1, 2]. The intense local inflammatory reaction within the small-bowel ultimately results in villous atrophy, crypt hyperplasia, and massive lymphocyte infiltration [3]. Malabsorption of micronutrients (e.g., vitamins and minerals) and macronutrients (e.g., protein, carbohydrate, fat) follows.

CD patients can be classified into three categories [4]: (a) symptomatic or classic, (b) atypical, and (c) asymptomatic or silent. The classic form occurs in infancy and manifests as a failure to thrive with diarrhoea, abdominal distension, and developmental delay. Failure to diagnose the disease may lead to a true medical emergency. The atypical form of CD is more frequent in adults and may present various non-specific symptoms (chronic fatigue, depression, fibromyalgia-like syndrome, aphthous stomatitis, bone pain, dyspepsia, gastroesophageal reflux) making the diagnosis quite challenging.

The treatment for CD appears relatively simple. A scrupulous life long diet free of gluten is required. For the majority of the patients, the diet will stop the symptoms, heal existing intestinal wound, and prevent further damage. Improvements begin within days of starting the diet. The small intestine is usually completely healed in 3 to 6 months in children and younger adults and within 2 years for older adults. In order to stay well, celiac patients must avoid gluten for the rest of their lives. This can be sometimes complex since gluten is found in most grain, pasta, cereal, and many processed foods. The help of a dietitian is often required at the beginning. However, as CD is more and more being acknowledged to be a frequent condition, gluten-free products are increasingly available in most regular shops.

Misdiagnosis of CD may have dramatic consequences on the long term. Calcium and vitamin D malabsorption dramatically increases the risk of osteoporosis and osteopenia in CD patients. Other autoimmune disorders have been shown to be associated with CD such as type I diabetes mellitus, autoimmune thyroid disease, autoimmune hepatitis amongst the most frequent. But the most feared complications are the development of either a specific enteropathy-associated T-cell lymphoma or a bowel adenocarcinoma. However, studies demonstrated that maintenance of a long-term gluten-free state reduces the risk of cancer to the level of the general population [5]. The impact of these complications is important for the patient, but also in terms of health costs. In an Italian study, each asymptomatic undiagnosed CD patient was estimated to cost the Italian Health Service up to 8'700 Euros per year due to the potential development of complications [6]. Despite the overall cost of screening, the saving for each patient was estimated up to 7'200 Euros. In a similar study in the USA, each diagnosed case of CD could save up to 7'400 USD per quality-adjusted life-year [7].

Traditionally thought to be a rare childhood disease, CD has now been recognized to be a frequent condition both in adults and children. Until recently, studies suggested that it affected one in 6'000 persons [8]. However, recent epidemiological studies have revealed that CD has been largely underdiagnosed and prevalence in many adult populations may be at least as high as 1:100 [9]. Moreover, CD affects populations worldwide (e.g., North and South America, Europe, North Africa, South and West Asia, Australia) and is not restricted to persons of European ancestry as initially thought [10, 11, 12].

Interestingly, CD is also a human leukocyte antigen (HLA) associated condition. With few exceptions, CD is limited to predisposed individuals who express the HLA-DQ2 (DQA1*0501/DQB1*0201), and those who express HLA-DQ8 (A1*0301/B1*0302) haplotypes [11]. Another common anomaly has been linked to the haplotype HLA-DQ2, selective IgA deficiency (IgAD) [13]. IgAD is the most common primary immunodeficiency and is defined by a serum IgA levels below 5 mg/dl with normal IgG and IgM and normal cell-mediated immunity [14]. As many as 20% of CD patients are estimated to have IgAD [15].

Current diagnostic criteria for CD are based on revised guidelines proposed by the European Society for Paediatric Gastroenterology and Nutrition [16]. According to these guidelines, intestinal biopsy is recommended as the gold standard for definitively ruling out the diagnosis of CD. However, this approach cannot be used as a screening procedure. Recently, many institutions are employing serological testing (detection of anti-gliadin antibodies, anti-endomysial IgA and anti-TGase2 IgA) as primary screening tools. Autoantibodies to TGase2 have been determined to be the most frequent antibodies associated to CD as described in a previous art [17]. Commercialized immunoassays based on TGase2 have sensitivities in excess of 90% and specificities over 95% [18]. However, these analyses are based on either ELISA or immunofluorescence technologies that both require well-equipped laboratories and qualified staff not always available worldwide as well as the repetition of the analysis for a single biological sample to identify different serological markers.

International Patent Application WO 2005/082254 [in the name of Ethicon, USA] disclosed two separate inventions. One of them concerns a diagnostic cap for liquid sample The diagnostic cap comprise a reaction zone with reagent moiety which reacts with an analyte and a detection zone with a detector moiety. The second part of the invention also concerns a diagnostic test apparatus comprising: a shaft having a first end and a second end; a swab or a biopsy punch mounted on the first end of a shaft; and a cap for fitting over the first end of the shaft, said cap containing at least one diagnostic test reagent; wherein the shaft comprises at least one cap engagement element located proximate to the first end, said element extending radially outwardly of the swab or the biopsy puch for engagement with the cap to retain the cap on the shaft. The configuration of the cap disclosed in this document is closed compared to the present invention. This document does not disclose an immunochromatography device and a method wherein the diffusion of the biological sample through the conjugate pads is carried out into at least three assay areas of the test pads.

Four one-step immunochromatographic assays have been previously described in WO 2005/082254 [in the name of Ethicon Inc, USA], International patent application WO95/04280 [in the name of Quidel Corp, USA], European Patent N° 1164375 [in the name of CT Genetica Biotech] and International Patent Application WO2004/016065 [in the name of Consejo Superior Investigation *et al*.].

International patent application WO95/04280 [in the name of Quidel Corp, USA] disclosed a one-step method of detecting analytes in a sample which require extraction prior to detection using test device. However, this document fails to disclose a device wherein the sample pad is adapted to direct at least part of said biological sample of a subject through conjugate pads into at least three assay areas.

European Patent N° 1164375 is based upon the detection of antibodies against TGase2 only as the total IgA level is not simultaneously detected with the detection of antibodies against TGase2 (with a sensitivity of IgA class antibodies against TGase 2 and with a sensitivity of IgG class antibodies against TGase 2) in the biological sample.

WO2004/016065 combines detection of both antibodies against TGase2 and gliadin. However, anti-gliadin antibodies have been found to be much less accurate than TGase2 and are therefore no longer recommended to be tested by the North American Society for Paediatric Gastroenterology, Hepatogy and Nutrition [21]. It has also been put forward in Lahteenoja *et al*., that salivary IgA or IgG directed against antigliadin are not helpful for the diagnosis or follow-up of celiac disease patients [35].

CD patients with IgAD will not have abnormally elevated levels of IgA against TGase2. This has led to the use of separate assays for total IgA and testing for IgG-class antibodies against TGase2 [22]. However, estimates of the sensitivity of IgG-class antibodies alone suggest that these tests have poor sensitivities around 40% [23].

Additionally, it is known from the abstract of Jurjus et al. [34] disclosed during the Experimental Biology 2005 Meeting (San Diego - March 31 to April 6, 2005) that saliva testing could be safely used as a non-invasive diagnostic and monitoring modality of Celiac disease except with patients with IgA deficiency.

Despite the disclosure of the foregoing patents and patent applications, there remains a need to develop a universal one-step screening method, easy to perform rapid and accurate, for simultaneously detecting the presence of total IgA and antibodies against TGase2 (IgA and IgG) in at risk CD populations amongst which those with IgAD.

This object has been achieved by providing an immunochromatography device for determining the presence of a binding analyte in a biological sample of a subject, comprising a sample application site for receiving said biological sample, conjugate pads allowing said binding analyte present in said biological sample to bind to a labeled agent, and test pads comprising assay areas, wherein the sample pad is adapted to direct at least part of said biological sample of a subject through said conjugate pads into at least three said assay areas.

### SUMMARY OF THE INVENTION

The present invention concerns an immunochromatography device for determining the presence of a binding analyte in a biological sample of a subject for simultaneously detecting the presence of total IgA and antibodies against TGase2 in at risk Celiac disease populations amongst which those with IgA deficiency, said binding analyte being an immunoglobulin selected from the group comprising an IgA, an sIgA, and IgG, or a combination thereof, comprising
a sample application site for receiving said biological sample,
conjugate pads allowing said binding analyte present in said biological sample to bind to a labeled agent, and test pads comprising assay areas,
wherein
i) a first conjugate pad is impregnated with a labeled anti human IgA and is in communication with a first test pad, said first test pad being coated, in the assay area, with tissue transglutaminase 2 (TGase2) or an antigenic part thereof and with anti human IgA or a binding portion thereof, and
ii) a second conjugate pad is impregnated with a labeled anti human IgG and is in communication with a second test pad, said second test pad being coated, in the assay area, with tissue transglutaminase 2 (TGase2) or an antigenic part thereof and with anti human IgG, a ubiquitous protein, or binding portions thereof,
and wherein the sample application site is adapted to direct at least part of said biological sample of a subject through said conjugate pads into at least three assay areas of said test pads and at least one of said test pads comprises two assay areas.

A further object of the present invention is to provide a method for determining the presence of a binding analyte in a biological sample of a subject for simultaneously detecting the presence of total IgA and antibodies against TGase2 in at risk Celiac disease populations amongst which those with IgA deficiency comprising the steps of
a) providing the immunochromatography device of any of claims 1-8,
b) collecting the biological sample,
c) pre-treating said biological sample in a pre-treating buffer,
d) contacting the pre-treated biological sample of step c) with the sample pad of said immunochromatography device, thereby enabling said pre-treated biological sample to diffuse laterally into at least three assay areas of said test pads,
e) running a lateral flow assay thereby allowing the sample to flow through the sample pad toward the conjugate pads and then toward the test pads, wherein said binding analyte, being an immunoglobulin selected from the group comprising an IgA, an sIgA, and IgG, or a combination thereof, if present in said biological sample, first contacts either anti-human IgA or anti-human IgG so that complexes with said anti-human IgA or anti-human IgG are formed and wherein said complexes further contact with tissue transglutaminase 2 (TGase2) or an antigenic part thereof,
f) enabling the development of a response and detecting the presence of the label present on the complexes,
interpreting the response to indicate the presence of said binding analyte in said biological sample.

Still another object of the invention is to provide a kit for determining the presence of a binding analyte in a biological sample of a subject, said kit comprising the immunochromatography device of the invention, optionally with reagents and/or instructions for use.

### DESCRIPTION OF THE FIGURES

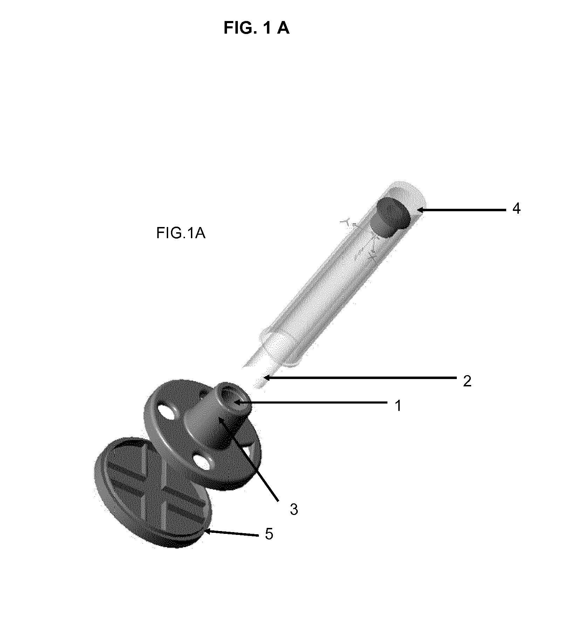

**Fig. 1A** shows an external perspective view of a preferred embodiment of the test device
**Fig. 1** **B** shows a side perspective view of one embodiment
**Fig. 2** shows the various test interpretation according to the visual results.
**Fig. 3** **A** shows an external perspective view of a second embodiment of the test device

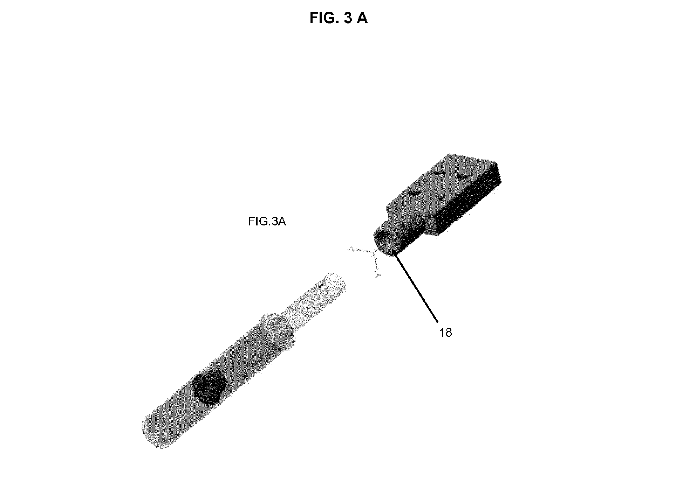

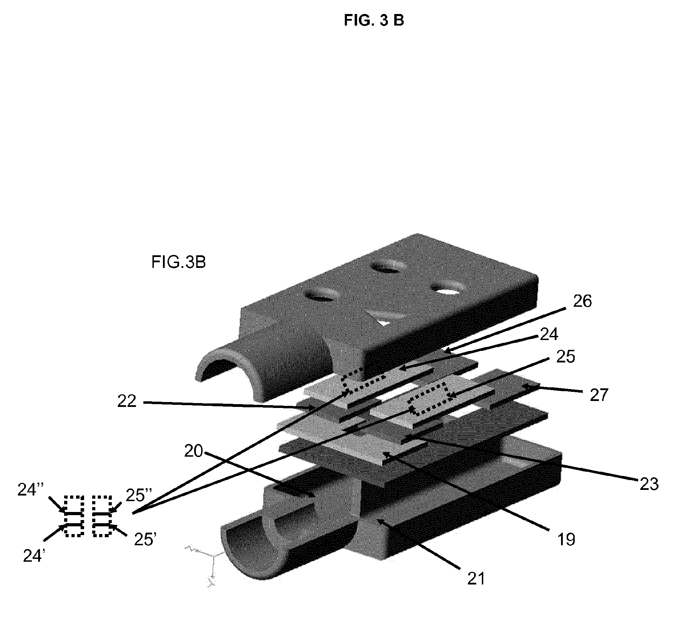

**Fig. 3** **B** details a side perspective view of a second embodiment of the test device

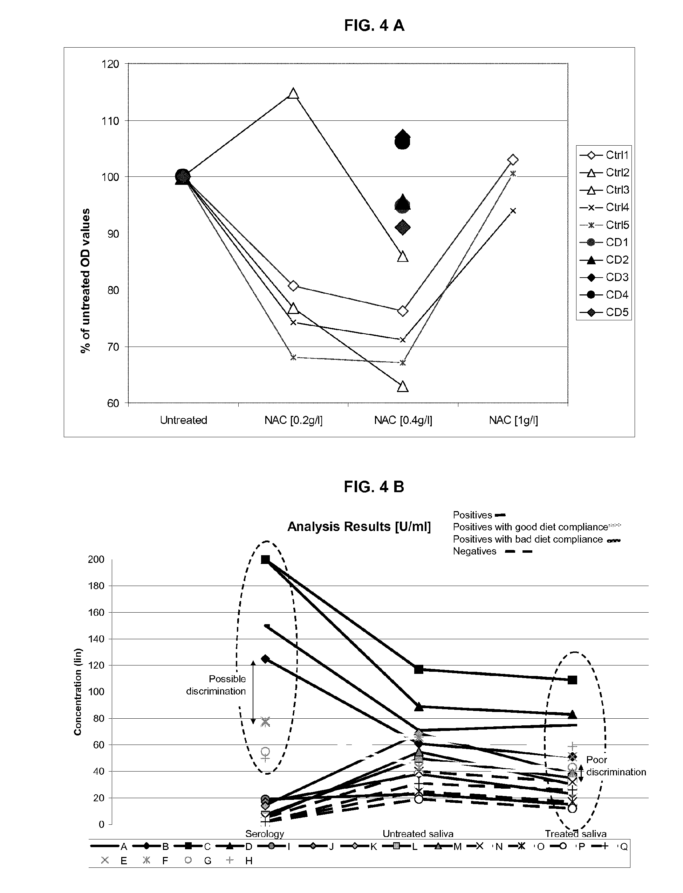

**Fig.** 4 **A** shows the effect of increasing concentration of mucolytic and reducing pre-treatment buffer on saliva samples
**Fig. 4** **B** details the IgA anti-TGase2 levels in serum, untreated whole saliva, treated whole saliva of patients with untreated CD, under bad diet compliance, under good diet compliance and of healthy control individuals as discussed in example 4.

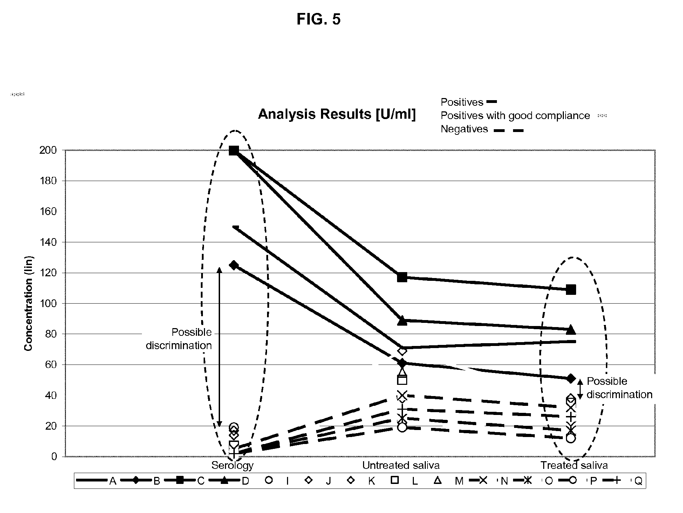

**Fig. 5** depicts the IgA anti-TGase2 levels in serum, untreated whole saliva, treated whole saliva of patients with untreated CD, under good diet compliance and of healthy control individuals.

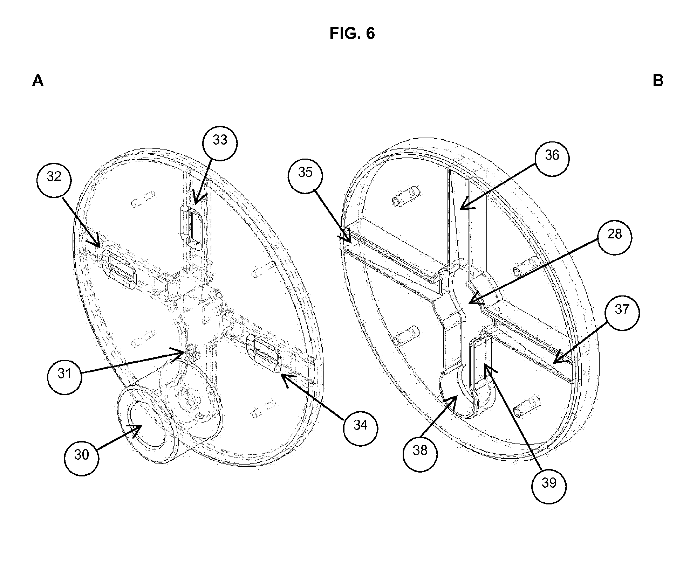

**Fig. 6** **A** shows an external perspective view (top part) of the third preferred embodiment of the test device
**Fig. 6** **B shows** an internal perspective view (bottom part) of the third preferred embodiment of the test device

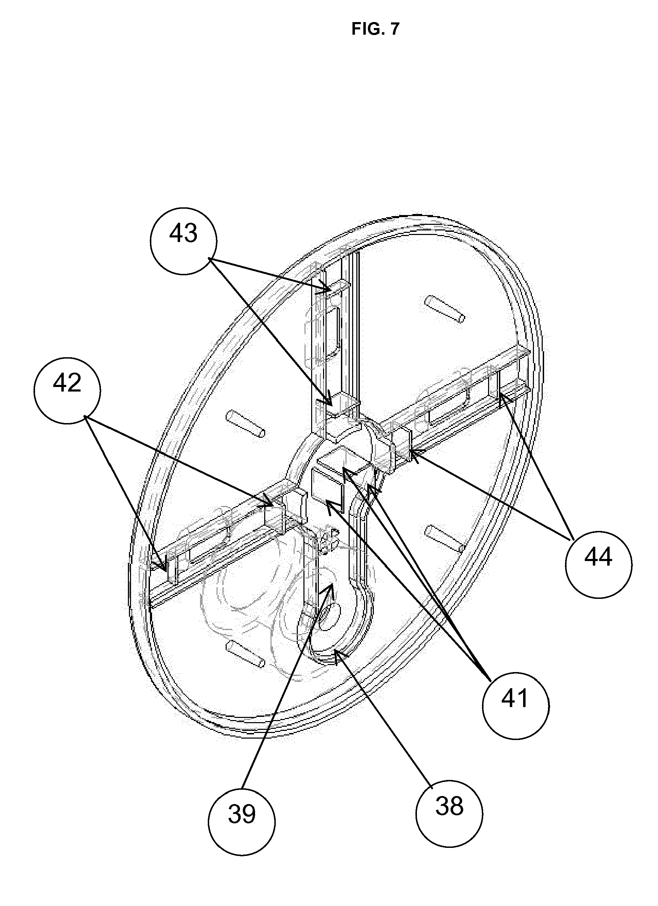

**Fig. 7** shows an internal perspective view (top part) of the third preferred embodiment of the test device

Other objects and advantages will become apparent to those skilled in the art from a review of the ensuing detailed description, which proceeds with reference to the following illustrative drawings, and the attendant claims.

### DETAILED DESCRIPTION OF THE INVENTION

The present invention concerns an immunochromatography device for determining the presence of a binding analyte in a biological sample of a subject for simultaneously detecting the presence of total IgA and antibodies against TGase2 in at risk Celiac disease populations amongst which those with IgA deficiency, said binding analyte being an immunoglobulin selected from the group comprising an IgA, an sIgA, and IgG, or a combination thereof, comprising a sample application site for receiving said biological sample, conjugate pads allowing said binding analyte present in said biological sample to bind to a labeled agent, and test pads comprising assay areas,
wherein
i) a first conjugate pad is impregnated with a labeled anti human IgA and is in communication with a first test pad, said first test pad being coated, in the assay area, with tissue transglutaminase 2 (TGase2) or an antigenic part thereof and with anti human IgA or a binding portion thereof, and
ii) a second conjugate pad is impregnated with a labeled anti human IgG and is in communication with a second test pad, said second test pad being coated, in the assay area, with tissue transglutaminase 2 (TGase2) or an antigenic part thereof and with anti human IgG, a ubiquitous protein, or binding portions thereof,
and wherein the sample application site is adapted to direct at least part of said biological sample of a subject through said conjugate pads into at least three assay areas of said test pads and at least one of said test pads comprises two assay areas.

"A" or "an" means "at least one" or "one or more."

"Immunochromatography device" usually refers to a membrane-based immuno lateral flow assay that allows visual detection of a binding analyte in liquid specimens.

A "binding analyte" as used herein, refers to a substance such as a chemical or a biological constituent that is undergoing analysis. Generally, the analyte may be a ligand or a binder. Usually, the binding analyte is an immunoglobulin (Ig). Preferably, the immunoglobulin is selected from the group comprising an IgA, a secretory IgA, or an IgG or a combination thereof. Most preferably, the binding analyte is an IgA and / or an IgG.

Typically, the immunochromatography device of the invention comprises a sample application site, conjugate pads, and test pads, said test pads comprising assay areas.

The "sample application site" as used herein, refers to a region dedicated for receiving the biological sample of the subject of the invention.

Preferably, the sample application site comprises at least one sample pad. A "sample pad" is capable of receiving a biological sample and filtering out, if necessary, any large particulate matter in the biological sample and holding the biological sample so that it can slowly wick into the assay. The sample pad is in lateral flow contact with the conjugate pads. This could either be an overlap or end-to-end connection. The sample pad is usually made of porous material such as cellulose, glass fiber, bonded glass fiber, and woven mesh. Preferably, the sample pad region is made of cellulose. Alternatively, the sample pad is impregnated with buffer to neutralize the extraction reagents during the lateral flow assay.

The term "lateral flow" refers to liquid flow in which all of the dissolved of dispersed components of the liquid are carried at substantially equal rates and with relatively unimpaired flow laterally through the material, as opposed to preferential retention of one or more components as would occur, e.g., in porous materials capable of adsorbing or imbibing one or more components.

As used herein, the terms "porous material" or "porous membrane" refer to any material capable of providing lateral flow. This would include material such as nitrocellulose, nitrocellulose blends with polyester or cellulose, untreated paper, porous paper, rayon, glass fiber, acrylonitrile copolymer or nylon. One skilled in the art will be aware of other porous materials that allow lateral flow.

As used herein, the term "subject" refers to any animal classified as a mammal, including humans, domestic and farm animals, and zoo, sports, or pet animals, such as dogs, horses, cats, cows, monkeys etc. Preferably, the mammal is human.

Usually, the biological sample of the invention is selected from the group comprising whole blood, serum, plasma, saliva, urine, brain tissue, and cerebral spinal fluid. Preferably, the biological sample is saliva.

The immunochromatography device of the invention further comprises conjugate pads which allow said binding analyte present in the biological sample to bind to a labelled agent. Usually, the conjugate pad is in lateral flow contact with the sample pad. This could either be by overlap or end-to-end connection. Alternatively, the conjugate pad may be on the same porous membrane as the sample pad. The conjugate pad is usually made of porous material such as cellulose, glass fiber, bonded glass fiber, and woven mesh. Preferably, the conjugate pad region is made of cellulose. Preferably also, the conjugate pads are impregnated, or associated, with said labeled (or labelled) agent. Alternatively, the labeled agent is bound to a microsphere. An example of such labeled microsphere is a dyed polystyrene microsphere.

Usually, the labelled agent is a monoclonal or polyclonal antibody. Preferably, it is selected from the group comprising anti human IgA, anti human secretory IgA, and anti human IgG. However, it is within the scope of the invention that the labelled agent may be any molecule with an affinity to a target binding analyte, including molecules that can bind to antigens, proteins, nucleic acids, cells, sub-cellular organelles, and other biological molecules. For example, the labelled agent may be derived from polyclonal or monoclonal antibody preparations, may be a human antibody, or may be a hybrid or chimeric antibody, such as a humanized antibody, an altered antibody, F(ab')2 fragments, F(ab) fragments, Fv fragments, a single-domain antibody, a dimeric or trimeric antibody fragment construct, a minibody, or functional fragments thereof which bind to the binding analyte of interest. Antibodies are produced using techniques well known to those of skill in the art. Furthermore, the labelled agent may be a receptor for a specific hormone or any other protein with specific binding affinities.

Attached to the labelled agent, either covalently or noncovalently, is a substance or particle capable of producing a signal (a label) detected visually. Such particles used as labeling reagents can be chromogens, catalysts, fluorescent compounds, colloidal metallic and nonmetallic particles, dye particles, enzymes or substrates, organic polymers, latex particles, liposomes with signal producing substances and the like.

Alternatively, the immunochromatographic device of the invention may be set up as a competitive design where the labeled agent is conjugated to a known amount of binding analyte and captured via an antibody or other compound with a specific affinity for the binding analyte.

Once the labeled agent has recognized and bound the binding analyte, a complex between these two entities is formed.

Each conjugate pad of the immunochromatography device of the invention is in communication with at least one test pad. The conjugate pad and the test pad may be present on a single porous membrane, or may comprise at least two separate porous membranes in lateral flow contact. The "test pad" of the invention is a membrane made of porous material which comprises a capture agent coated in the test area. Preferably, the capture agent is applied and dried onto the substrate which composes the test pad. The capture agent is not affected by the lateral flow of the biological sample due to the immobilization to the porous material. The capture agent is capable of "capturing" the complex formed between the binding analyte and the labeled agent when the complex present in the biological sample flows past the coated capture agent and where it immobilizes.

As used herein, "in communication" means that the different pads are in contact with each other by either overlap, continuation or end-to-end connection so as to ensure the flow to diffuse throughout the immunochromatographic device of the invention.

Usually, the test pad of the invention comprises assay areas. As used herein, "an assay area" refers to an area that delimits a region on the test pad where at least one capture agent is applied and visualized onto the substrate which composes the test pad. Usually, the assay area can be viewed through, in case the device comprises a plastic cover, a window placed on said plastic cover.

Preferably, one of said test pads of the invention comprises two assay areas or, alternatively, each of said test pads comprises one assay area.

The immunochromatography device of the invention is characterized in that the sample application site is adapted to direct at least part of said biological sample of a subject through said conjugate pads into at least three assay areas of said test pads. This is enabled by the fact that the sample application site has a configuration that helps the diffusion of the biological sample, for example by being in close contact with the conjugate pads.

The number of assay areas available in the immunochromatography device is comprised between 3 and 12, preferably between 4 and 10, more preferably between 4 and 8. The inferior limit of assay areas has been determined in the order of 3 in accordance with example 1 illustrating a preferred embodiment. In this case, a first assay area (15) reveals total IgA in the biological sample, a second assay area (16) reveals the presence of IgA against TGase 2 whereas a third assay area (17) reveals the presence of IgG against TGase 2. Surprisingly, applicants have shown that these three assay areas are mandatory, in the case the disease to be detected is CD, for detecting CD in at risk populations amongst those with IgAD. Preferably, the immunochromatography device of the invention comprises one additional assay area which corresponds to an internal control which serves to validate the assay.

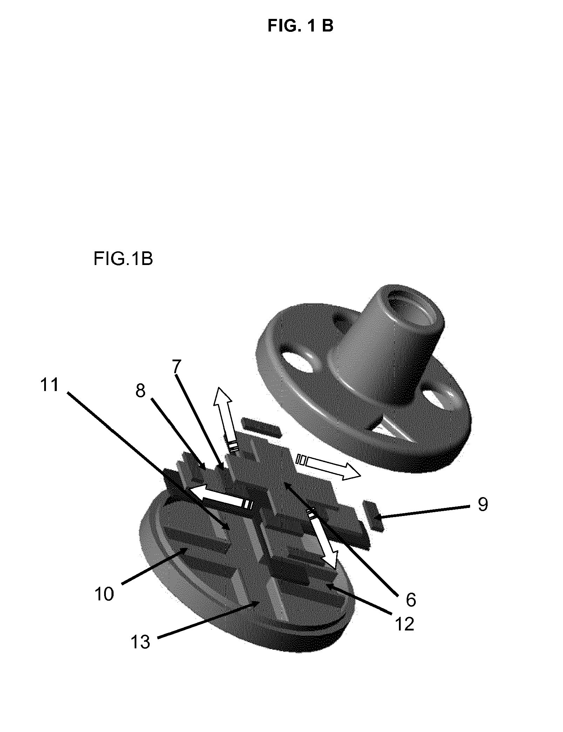

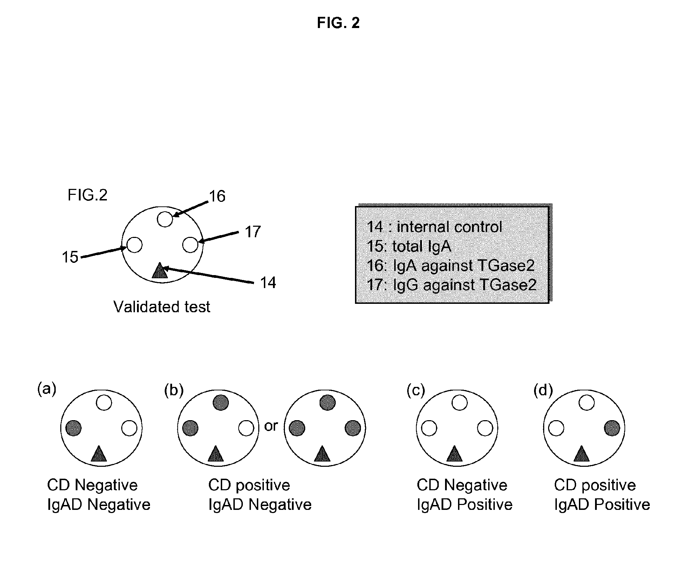

Referring in more details to Figs 1A and 1B, the sample pad (6) of the immunochromatography device of the invention has a cross shape and is in contact by overlapping the conjugate pads (7) within the 4 lanes (10), (11), (12), and (13). Each conjugate pad (7) is partly overlapping a test pad (8). The assay areas correspond to the 4 regions (14), (15), (16) and (17) as represented in Fig. 2. A plastic material covers the porous material of the immunochromatographic device.

Another preferred embodiment is represented in Figs 3A and 3B. In this specific case, the sample pad (19) has a U-shape and is overlapped by two conjugate pads (22, 23). Each of the conjugate pads (22, 23) is overlapped by one test pad (24, 25). These test pads (24, 25) comprise each an assay area (24', 24", 25', 25") which can be viewed through, for example, a window placed on the plastic cover.

In a third preferred embodiment represented in Figs 6 and 7, the sample application site is also de-centered with regard to the sample pads. Optionally, this sample application site may also function as a pre-treatment chamber where the biological sample is contacted with a pre-treatment solution.

Once the biological sample is applied to the sample application site, then it gentle flows through a channel from the sample application site towards a sample receptacle. Usually, the flowing movement of the biological sample is caused for example by gravitational forces or by gentle diffusion. To avoid flooding, the device may contain a window for air flow (31). Then the sample pads are contacted with the biological sample by plunging into the sample receptacle. Preferably, each of the sample pads is mounted on a lane (35, 36, 37) which maintains the sample pad into the sample receptacle (28). Additionally, there are means, such as three walls, see Fig. 7 (41), insuring that all sample pads plunge into the biological sample. Each sample pad is then in communication with at least one conjugate pad. Preferably there are also walls (42, 43, 44) maintaining pressure at the interface between the components of each one of the lateral flow test strips. The lane is usually in a horizontal position or, preferably slightly inclined with an inclination angle between 0-20°, preferably an inclination of 10°, that insures that there is no overflooding of the test strips.

The conjugate pad and the sample pad may be present on a single porous membrane, or may comprise at least two separate porous membranes in lateral flow contact. Usually, the different pads are in contact with each other by either overlap or end-to-end connection so as to ensure the flow to diffuse throughout the immunochromatographic device of the invention.

Usually, a plastic material covers the porous material of this immunochromatographic device. Each test pad, which is in contact with the conjugate pad by either overlap or end-tend connection is also mounted on a lane (35, 36, 37) and comprise each an assay area which can be viewed through, for example, a window placed on the plastic cover (32, 33, 34). Figure 7 represents an example of this third preferred embodiment comprising three lanes (35, 36, 37) each comprising an assay area (32, 33, 34).

In the example of a preferred embodiment, the device comprises 3 lanes (35, 36, 37) arranged around the center of a circle like the spoke of a wheel wherein the center constitute a sample receptacle (28). The sample application site (38) is de-centered with respect to the lanes and is linked to the sample receptacle through a little channel (39). The sample application site may be designated (30) as to adapt to saliva collector device such as Omni-SAL^{®}.

However, it is also envisioned to have a device with only two lanes or more than three and comprising three or more assay areas.

This device presents several manufacturing advantages such as
- ease to assemble the tests strip within the device,
- the sample pre-treatment step can be performed within the device and
- there is no risk of test strip flooding while applying the biological sample.

Preferably, at least one test pad of the immunochromatophy device of the invention is a control pad. This control pad serves to validate the assay and comprises a test area which comprises an immobile capture agent acting as an internal control. Usually, the capture agent which is coated in the test area of said control pad will recognize a complex formed between labeled agent and a binding analyte which is ubiquitously present in the biological sample of the subject, regardless of the presence or absence of the binding analyte of interest in the biological sample. Once it binds the capture agent present on the control pad it immobilizes the complex and prevents it from continuing lateral flow with the liquid sample. The capture agent for the control pad may be applied to the porous membrane in any geometrical shape desired. In the event that the development of a response is not positive, the assay is not valid and it must be repeated using a new immunochromatography device of the invention.

In case the binding analyte that is undergoing analysis is present, or susceptible to be present, in saliva, then it is also envisioned that the device of the invention further comprises a recipient for a saliva collector device such as a cotton pad device. Preferably, the saliva collector device is the Omni-SAL^{®} collector (Saliva Diagnostic Systems Inc., Brooklyn, New York, USA).

The device of the invention may also comprise soak pads, located at the distal end of each test pad. The soak pad may be on the same porous membrane as the test pad, or may be a separate porous membrane in lateral flow contact with the test pad. The soak pad is capable of absorbing and holding the biological sample after it has wicked across the assay, preventing the sample from flowing in the opposite direction, and causing non-specific binding to occur. The contact with the test pad can be either by overlap or end-to-end connection. Alternatively, the soak pad may be on the same porous membrane as the test pad. The soak pad is made of porous material, usually nitrocellulose filters.

Usually, the binding analyte must be capable of migrating through lateral flow with the biological sample. If it is not the case, pre-treatment of the biological sample may help promoting the biological sample solubilisation and reducing non-specific binding.

The sample pad, the conjugate pad, the test pad and the soak pad can be made of different material, or can be separate regions of the same porous membrane. The test pad can contain the mobile labeling agents, the immobile capture agent and the immobile control capture agent. In other embodiments the analyte detection region contains only the immobilized control capture reagent and the capture reagent.

Additionally, the sample application site of the immunochromatography device of the invention may comprise means for maintaining in contact the at least one sample pad with the biological sample of a subject. As used herein, the term "means" refer to any alternative enabling, maintaining or favoring the contact between the sample pad and the biological sample. Non limiting examples of means are bridges and walls.

In a further embodiment, a first conjugate pad of the immunochromatography device of the invention is impregnated with a labeled anti human IgA and a second conjugate pad is impregnated with a labeled anti human IgG. In such as case, said first conjugate pad impregnated with a labeled anti human IgA is in communication with a first test pad, said first test pad being coated, in the assay area, with tissue transglutaminase 2 (TGase2) or an antigenic part thereof and with anti human IgA or a binding portion thereof, and said second conjugate pad impregnated with a labeled anti human IgG is in communication with a second test pad, said second test pad being coated, in the assay area, with tissue transglutaminase 2 (TGase2) or an antigenic part thereof and with anti human IgG, a ubiquitous protein, or binding portions thereof.

Usually, a semi-rigid material will be used to support the porous material(s) of the immunochromatography device of the invention. This semi-rigid material can be one continuous piece of laminate or separate pieces. The laminate is preferably vinyl but one skilled in the art will recognize that numerous materials can be used to provide the semi-rigid support. A minimal thickness is required in order to produce the desired adequate mechanical strength or support for the immunochromatography device to function effectively such as to provide sufficient strength and support to the porous material and assay device such that no interference with the lateral flow results, for instance from the collapse or disintegration of the immunochromatography device upon wetting.

Any plastic material can cover the porous material of the device. Preferably, this plastic is mylar, however, those skilled in the art will know of various materials that can be used for such purposes. The cover can be one continuous plastic or separate pieces as shown in figures 1 and 3. It must allow the assay areas to be viewed. Thus, if the cover is clear then the result can be viewed through the clear cover. If the cover is not clear, then a window, gap or hole must be used so the results can be viewed. In addition, the cover can leave a portion of the sample recipient exposed so the sample can be applied to the receiving region in a region called the sample application site (for example, references (1), fig. 1A and (18), fig. 3).

Alternatively, the backing and plastic cover can be a molded plastic housing. Preferably, this plastic is a polyester, however, those skilled in the art will know of various materials that can be used for such purposes.

Encompassed in the present invention is the use of the immunochromatography device as described herein for determining the presence of a binding analyte in a biological sample of a subject.

Also encompassed by the present invention is a method for determining the presence of a binding analyte in a biological sample of a subject for simultaneously detecting the presence of total IgA and antibodies against TGase2 in at risk Celiac disease populations amongst which those with IgA deficiency comprising the steps of
a) providing the immunochromatography device of the invention,
b) collecting the biological sample,
c) pre-treating said biological sample in a pre-treating buffer,
d) contacting the pre-treated biological sample of step c) with the sample pad of said immunochromatography device, thereby enabling said pre-treated biological sample to diffuse laterally into at least three assay areas,
e) running a lateral flow assay thereby allowing the sample to flow through the sample pad toward the conjugate pads and then toward the test pads, wherein said binding analyte, being an immunoglobulin selected from the group comprising an IgA, an sIgA, and IgG, or a combination thereof, if present in said biological sample, first contacts either anti-human IgA or anti-human IgG so that complexes with said anti-human IgA or anti-human IgG are formed and wherein said complexes further contact with tissue transglutaminase 2 (TGase2) or an antigenic part thereof,
f) enabling the development of a response and detecting the presence of the label present on the complexes,
g) interpreting the response to indicate the presence of said binding analyte in said biological sample.

There is no particular limitation on the methods for collecting biological sample of a subject. For example, whole blood drop is collected from a fingertip using a lancet device and diluted in the pre-treatment buffer described infra for direct test application, or stored in heparin-coated capillary tubes prior to testing. Specifically, saliva is collected using cotton pad devices such as Omni SAL ® (Saliva Diagnostic Systems Inc., Brooklyn, New York, USA) [US patent N. 5,260,031], OraSure® HIV-1 oral fluid specimen device and UpLink® (OraSure Technologies Inc., Bethlehem, USA), Salivette® (Sarstedt, Newton, N.C., USA), TRANSORB® wicks (Filtrona Richmond Inc., Colonial Heights, Vancouver, USA). The pad is then placed in the pre-treatment buffer as described in the examples.

Preferably, the method of the invention is designed for the diagnosis of celiac disease, , in particular in populations with IgAD.

The biological samples such as whole blood, serum or saliva are diluted into pre-treatment buffer as described in the examples. A preservative agent may be added to the pre-treatment buffer.

In case the biological sample is saliva, the pre-treatment solution also allows processing saliva samples to enhance detection of, for example, IgA against TGase2. Interactions of mucins and IgA favour non-specific staining and false-positive results. As known in the art, mucolytics or reducing agents may be added to the pre-treatment solution to cleave chemical bonds between mucins and IgA [24].

The method of the invention further comprises the step of contacting the pre-treated biological sample with the sample pad of the immunochromatography device of the invention, thereby enabling said pre-treated biological sample to diffuse laterally into at least three assay areas.

Typically, the pre-treated biological sample flows through the sample pad toward the conjugate pads and then toward the test pads comprising the assay areas. When the binding analyte is present in said biological sample, it first contacts labeled agents impregnated on each conjugate pad so that complexes with said labeled agents impregnated on conjugate pads are formed.

As described supra, the binding analyte is an immunoglobulin and is selected from the group comprising an IgA, or an IgG or a combination thereof. Preferably, when the immunochromatography device is used for the diagnosis of a celiac disease, the binding analyte is an IgA and /or an IgG.

Also usually, the labeled agent, designed to recognize, bind and form a complex with the binding analyte, is selected from the group comprising anti human IgA, anti human IgG,. In case the binding analyte is IgA and /or an IgG, then the labeled agent is an anti human IgA or an anti human IgG made, for example, in goat.

Preferably, the label which is associated with the agent is selected from the group comprising, but not limited to, enzymes, dyes, and visible particles.

Once the complexes are formed, they further contact with capture agents or antigenic parts thereof which are coated, in the assay area, on test pads. The capture agent is capable of "capturing" the complexes when present in the biological sample. Usually, the capture agent is any particle or molecule such as a protein which recognizes or binds the complex with the labeling agent that has bound to the biological analyte in the sample. The capture agent is immobilized to the porous material of the test pads, more particularly located in the assay area. The capture agent is not affected by the lateral flow of the liquid sample due to the immobilization to the porous material. The capture agent can be natural, or non-natural, i.e., synthetic. Once the capture agent binds the analyte-labeled agent complex it prevents the complex from continuing with the lateral flow of the liquid sample.

The method of the invention further comprises the steps of enabling the development of a response and detecting the presence of the label present on the complexes. Generally, the reaction is visualized through the deposition of a colored complex.

In case the label is an enzyme, then the addition of, for example, a colored substrate specific to the enzyme will enable the visualization of the complex.

Usually, the method of the invention comprises the step of interpreting the response to indicate the presence of said binding analyte in said biological sample. Generally, a visual examination of the assay area after 10-20 minutes is sufficient to interpret the results of the test. Referring in more details to the preferred embodiment (example 2, Fig. 2), the immunochromatography device must be positioned with the window corresponding to the assay area (14), which corresponds to the internal control, in front of the user. The test is validated if a blue line appears in this window. A total of 5 combinations may be visualized leading to 4 distinct clinical diagnostics as described in Fig. 2 and Table 1.

**Table 1**

| Assay areas | | | | Interpretation |
|---|---|---|---|---|
| (14) | (15) | (16) | (17) | |
| + | + | - | - | Internal control shows that the test is validated, the subject is neither IgA deficient nor suffering from CD. |
| + | + | + | - | Internal control shows that the test is validated, the subject is not IgA deficient but is suffering from CD. |
| + | + | + | + | |
| + | - | - | - | Internal control shows that the test is validated, the subject is IgA deficient but is not suffering from CD. |
| + | - | - | + | Internal control shows that the test is validated, the subject is IgA deficient but is suffering from CD. |

Preferably, the pre-treating buffer is selected from the group comprising N-acetyl-cystein in PBS buffer or carbocystein min in PBS buffer, optionally with a reducing agent such as DDT or SDS. Also optionally, a preservative agent such as EDTA or sodium azide may be added to the pre-treating buffer. It is in the capacity of one skilled in the art to find out the best pre-treating buffer according to the biological sample.

Usually, the label is selected from the group comprising chromogens, catalysts, fluorescent compounds, colloidal metallic and nonmetallic particles, dye particles, enzymes or substrates, organic polymers, latex particles, liposomes with signal producing substances and the like.

The one-step method is dedicated for the detection of total IgA simultaneously to the detection of antibodies against TGase 2 (with a sensitivity of IgA class antibodies against TGase 2 and with a sensitivity of IgG class antibodies against TGase 2) in the biological sample.

Also with in the scope of the present invention is a kit for determining the presence of a binding analyte in a biological sample of a subject, said kit comprising the immunochromatography device as described herein, optionally with reagents and/or instructions for use.

The kit of the present invention may further comprise a saliva collector device.

Alternatively, or additionally, the kit may further include other materials desirable from a commercial and user standpoint, including buffers, diluents, filters, needles, and syringes, for example for collecting the biological sample.

The kit may also include a system enabling the detection of the response. The response may be detected visually or instrumentally depending on the label present on the complexes. Possible responses may include production of coloured, fluorescent, or luminescent products, alteration of the characteristics of absorption or emission of radiation by an assay component or product, and precipitation or agglutination of a component product. Said component may be a label, *e.g.* a radioisotope, a fluorophore, an enzyme, a co-enzyme, an enzyme substrate, an electron-dense compound, or an agglutinable particle.

The foregoing description will be more fully understood with reference to the following Examples. Such Examples, are, however, exemplary of methods of practising the present invention and are not intended to limit the scope of the invention.

### EXAMPLES

### Example 1:

### Simple Collection

Preferably, the samples are, for example, whole blood, serum or saliva. There is no particular limitation either on the methods for collecting these samples. For example, whole blood drop is collected from a fingertip using a lancet device and diluted in the pre-treatment buffer described below for direct test application, or stored in heparin-coated capillary tubes prior to testing. Specifically, saliva is collected using cotton pad devices such as Omni SAL ® (Saliva Diagnostic Systems Inc., Brooklyn, New York, USA) [US patent N. 5,260,031], OraSure® HIV-1 oral fluid specimen device and UpLink® (OraSure Technologies Inc., Bethlehem, USA), Salivette® (Sarstedt, Newton, N.C., USA), TRANSORB® wicks (Filtrona Richmond Inc., Colonial Heights, Vancouver, USA). For example, the Omni-SAL® collector pad is placed under the tongue until an indicator turns blue signalling that 1ml saliva has been collected. The pad is placed in the pre-treatment buffer described below and can be either stored at 4°C prior to testing or is extracted using an adapted filter for immediate test application.

### Simple Processing

The biological samples (whole blood, serum or saliva) are diluted into 1ml of pre-treatment buffer with the following composition: Tris-buffered-saline (TBS) at a pH of 7.3 containing a non-ionic detergent such as Tween® 20 or Triton X-100® at a concentration of 0.3%. A preservative agent maybe used such as sodium azide or ethylene-diamine-tetraacetic acid (EDTA). The pre-treatment solution also allows processing saliva samples to enhance detection of IgA against TGase2. Interactions of mucins and IgA favour non-specific staining and false-positive results. As known in the art, mucolytics such as N-acetyl-cystein or carbocystein may be added to the pre-treatment solution to cleave chemical bonds between mucins and IgA [24]. The addition of reducing agents such as dithiothreitol (DTT) or sodium dodecyl sulphate (SDS) at low concentrations (between 0.5 to 2.5 mM) has the same effect by splitting disulfide bonds while preserving the antigen binding ability of immunoglobulins required for an immunoassay [25, 26].

### Example 2:

Two preferred forms of embodiments of the invention will be illustrated as examples in the drawings and described in details herein, albeit different embodiments are possible. Moreover, both forms of embodiments are designed to adapt to existing saliva collectors. The specific focus on salivary specimens is due to the non-invasive feature of saliva collection most suitable for screening purposes, especially within the paediatric population.

In Fig.1A, the test device has a circular configuration and as defined allows simultaneous targeted analysis. The site of the specimen application (1) is designed as to adapt to saliva collector devices such as Omni-SAL®. The end part of the filter (2) is inserted into the sample recipient (3). Pushing the collector (4) towards the test device (5) allows filtering of the specimen that flows onto the sample pad (6) placed at the centre of the test device, as shown on Fig.1B.

Other specimens can be directly loaded on the sample pad using pipettors or similar devices. The sample pad is specified as to allow a large bed volume (> 25µl/cm²) and is also used to load low concentrations of bovine serum albumin (BSA) and surfactants such as Tween® 20 to promote resolubilization of the labelled secondary antibodies and reduce non-specific binding. The sample is then evenly distributed to the conjugate pads (7, Fig.1B) promoting reconstitution of labelled secondary antibodies. The conjugate pad is also specified as to hold high bed volume. For both sample and conjugate pad, different materials with absorbent properties may be used such as cellulose, glass fiber, bonded glass fiber, and woven mesh, but preferentially cellulose filters. All these materials allow the sample deposited at one end of the assay or test strip to migrate and to diffuse laterally at the opposite end by capillary action. Two different labelled monoclonal antihuman antibodies, anti-IgA and anti-IgG are immobilized on the different conjugate pads. The binding analyte, if present in the biological sample, first contacts labelled agents such as the labeled secondary antibodies impregnated on each conjugate pad so that a complexes with labelled secondary antibodies migrates are formed and said complexes further laterally migrate into the test pads (8) (as demonstrated by the arrows, Fig.1B) where the specific capture agent or an antigenic part thereof is immobilized. Different membrane polymers may be used to constitute the test pads such as nitrocellulose, polyvinylidene fluoride, charge-modified nylon or polyethersulfone, but preferentially nitrocellulose membranes with a pore size of 0.4µm. As an example, the High-Flow Plus Membranes® (Millipore, Bedford, Massachusetts, USA) with a capillary flow time ranging from 120 to 180 sec/4cm meet the designed test requirements.

Soak pads (9) composed from nitrocellulose filters are located at the distal ends of the of each test pad to contain excessive fluid.

Fig.1B details the test strip assembly within the circular configuration embodiment. The sample pad (6) has a cross shape and overlaps the conjugate pads (7) within the 4 lanes (10), (11), (12), and (13). In lane (10) and (11), labelled anti-human IgA is immobilized in the conjugate pad. Labelled anti-human IgG is immobilized in the conjugate pad of lane (12). Lane (13) contains the necessary elements for control of the immunochromatographic conditions. Each lane corresponds to a specific *target marker* to be revealed on the nitrocellulose membrane such as:
- lane 10 anti-human IgA reveals total IgA in the biological sample;
- lane 11 TGase2 reveals the presence of IgA against TGase2 in the biological sample;
- lane 12 TGase2 reveals the presence of IgG against TGase2 in the biological sample;
- lane 13 validates the test.

Fig.2 shows the various test interpretation according to the visual results. To read the results, the test device is positioned with the triangle window (assay area (14)) in front of the user. This window corresponds to the internal control. A blue line within this window needs to be visualized for the test to be validated. If no line appears, the test failed and needs to be repeated, if necessary with another immunochromatography device. The other windows give the following results:
- assay area (15): total IgA
- assay area (16): IgA against TGase2
- assay area (17): IgG against TGase2

A total of 5 combinations may be visualized leading to 4 distinct clinical diagnostics as described on Fig.2.

Fig.3A shows another form of embodiment for the invention. The sample recipient (18) is also designed as to adapt to saliva collector such as Omni-SAL®, but other types of specimens may be directly loaded on the sample pad using pipettors or similar devices. As detailed on Fig.3B, the sample pad (19) has a horseshoe shape. The biological sample migrates laterally along both channels (20), (21), and proceeds through the conjugate pads containing labelled anti-human IgA (22) on one side and labelled anti-human IgG (23) on the other side. On a first membrane (24), two labelled agents are immobilized, anti-human IgA for total IgA detection, and TGase2 for revealing the presence of IgA against TGase2. To gain a higher sensitivity, TGase2 is placed downstream of anti-human IgA. On a second membrane (25), TGase2 as well as downstream all the necessary elements for an internal control of the immunochromatographic conditions are deposited. Soak pads (26), (27) are placed at both ends of test or assays strips. The results are visualized in the same way as described above leading to 5 possible combinations and 4 distinct clinical diagnostics.

Both test devices illustrated and described above meet the necessary requirements for a universal screening tool allowing the detection of CD in all at risk groups without the need to perform additional tests. The test device as described herein in significantly less expensive than actual laboratory ELISA assays. It can be used on different biological samples, but preferentially on saliva owing to the non-invasive collection methods. As such, the device adapts to existing saliva collectors. The analysis is a simple one-step procedure yielding rapid results.

### Example 3:

### Simple Collection

There is no particular limitation as to the types of biological samples to be tested. Preferably, the samples are, for example, whole blood, serum or saliva. There is no particular limitation either on the methods for collecting these samples. For example, whole blood drop can be collected from a fingertip using a lancet device and diluted in the pre-treatment buffer described below for direct test application, or stored in heparin-coated capillary tubes prior to testing. Specifically, saliva may be collected using cotton pad devices such as Omni SAL ® (Saliva Diagnostic Systems Inc., Brooklyn, New York, USA) [US patent #5,260,031], OraSure® HIV-1 oral fluid specimen device and UpLink® (OraSure Technologies Inc., Bethlehem, USA), Salivette® (Sarstedt, Newton, N.C., USA), TRANSORB® wicks (Filtrona Richmond Inc., Colonial Heights, Vancouver, USA). As an example, the Omni-SAL® collector pad is placed under the tongue until an indicator turns blue signaling that 1ml saliva has been collected. The pad is placed in the pre-treatment buffer described below and can be either stored at 4°C prior to testing or is extracted using an adapted filter for immediate test application.

### Simple Processing

Sample pre-treatment solution is adapted to suit all types of biological samples, but applies preferentially to whole blood, serum and saliva.

The biological samples (whole blood, serum or saliva) are diluted into 1ml of pre-treatment buffer with the following composition: Tris-buffered-saline (TBS) at a pH of 7.3 containing a non-ionic detergent such as Tween® 20 or Triton X-100® at a concentration of 0.3%. A preservative agent may be used such as sodium azide or ethylene-diamine-tetraacetic acid (EDTA).

### Example 4:

Although the following example assays have been performed by ELISA, they fully confirm the results obtained with the device of the invention.

### Subjects

The goal of this clinical study was to validate the use of the marker combination in both saliva and serum. The samples were first tested on an ELISA platform prior to further testing on lateral flow tests. The clinical study was designed to collect saliva samples from patients consulting at the Gastroenterology Division of the Pediatric Department at the University Hospital of Lausanne (CHUV) in Switzerland. Informed consent was obtained from all subjects under study. The study was approved by the applicable institutional review board. A total of 30 patients were enrolled in the study. The subjects were classified into 6 groups. Group I comprised 4 untreated CD patients (2 males and 2 females; median age 11 years; range 7-15 years) diagnosed in 2007 according to the ESPGHAN criteria. Two of the patients had an atypical CD presentation with isolated growth failures. Another one had a silent CD form in the context of a diabetes type I. The last one suffered from a CD with a classic presentation.

Group II was composed of 6 diagnosed CD patients (6 females; median age 23 years; range 18-34 years) under a poorly controlled gluten-free diet.

Group III comprised 5 diagnosed CD patients (4 females and 1 male; median age 18 years; range 6-18 years) under a strict gluten-free diet for at least 6 months.

Group IV was composed of 3 IgAD patients amongst which there was 2 CD patients (1 female and 2 males; median age 9 years; range 9 years).

Group V comprised 6 healthy controls (2 females and 4 males; median age years; range years). None of them suffered from another autoimmune disease.

### Serum Specimens

Blood was collected by venipuncture and serum extracted by centrifugation. Serum samples were diluted 1:101 as described by the manufacturer's instructions (QUANTA Lite™ h-tTG IgA and IgG from Inova Diagnostics Inc.,San Diego, CA, USA).

### Saliva Collection Method

Saliva samples were collected in all patients using the OmniSAL™ collector device (StatSure Inc., Framingham, MA, USA). This device consisted of a cellulose pad attached to a plastic stem containing an indicator dye. The pad was placed under the patient's tongue. The dye turned blue when 1ml of saliva had been collected. The pad was then removed from the stem by manual twisting in a transport tube containing a modified phosphate buffer with sodium azide and Tween 20 (0.2%) at physiological pH (StatSure Inc., Framingham, MA, USA). The samples were immediately stored at -804°C and thawed only once prior to testing.

### Saliva Extraction and Processing

Prior to processing and testing, saliva was extracted from the collector pad by using a piston-style filter which was manually inserted into the transport tube. About 1ml of a clear solution consisting of approximately equal volumes of saliva and buffer was obtained in the filter. Sterile demineralized aqueous solutions containing different concentrations of N-acetyl-cystein (Sigma Aldrich Grade A7250) 10g/L, 20g/L and 50g/L were prepared. 5µl of each one these solutions were added to 100µl of extracted sample with final concentrations of N-acetyl-cystein (NAC) varying from 0.2 to 2.4 g/L of saliva. Samples were then incubated at room temperature for 20 minutes. Saliva samples were either used pure or diluted 1:10 prior to testing.

### Detection of Anti-TGase2 Antibodies and total IgA

ELISA assays were performed to detect IgA and IgG anti-TGase2 antibodies. A commercial kit, QUANTA Lite™ h-tTG IgA and IgG from Inova Diagnostics Inc. (San Diego, CA, USA) was used. These tests were performed on both serum and saliva samples for all patients, according to the manufacturer's instructions for use. Total IgA were measured in serum using and ELISA technology.

### Results

Antibody concentration in serum is 10 times superior (IgA in the range of 75 to 300 µg/ml) than in saliva (IgA in the range of 30 ± 15 µg/ml). However, a 1:10 dilution factor had a too strong impact on test sensitivity yielding very low absorbance measures. All further assays were performed using pure saliva.

Saliva is a heterogeneous medium with viscosity properties due to the presence of mucins. The latter are amongst the largest glycosylated proteins (2 to 50 MDa) that are secreted on the mucosal surfaces to protect cells [4]. They have the particularity to form aggregates and complexes with other polymers, and therefore induce a strong non-specific background staining on immunoassays [5]. Moreover, antibodies are trapped within these mucin filaments inducing a loss of signal for the immunoassay. The need for a mucolytic and reducing pre-treatment (combination of NAC and Tween 20) of the saliva samples has several advantages. It allows degrading mucin aggregates to render the sample more fluid for lateral flow migration. It also induces cleavage of chemical bonds between mucins and immunoglobulins. This effect is illustrated on Figure 4A.

At a concentration of 0.4g/l of NAC and 0.2% Tween 20, the negative controls showed the lowest OD values. In comparison, saliva samples from CD patients treated using the same buffer concentrations yielded no significant differences with OD values of untreated samples. The main effect of the pre-treatment buffer was to avoid non-specific background staining from mucins and therefore increase discrimination between the positive and negative populations.

Using the optimal concentration of mucolytic and reducing agents that lowered the most the signal for the negative controls, the different groups of patients were assessed for IgA and IgG anti-TGase2. The results are shown on Figure 4B. When considering all the Groups of patients, it is difficult to discriminate the different populations. There are major overlaps between Group I (untreated CD), II (bad diet compliance) and III (good diet compliance). When eliminating Group II, discrimination is made possible between the other populations, as shown on Figure 5. These preliminary results demonstrate that it is feasible to use saliva to distinguish between an untreated CD patient and a healthy individual. Therefore, saliva could be an excellent non-invasive screening medium. However, when considering follow-up of CD patients under diet, it becomes very difficult to discriminate the good from the bad diet compliances. For this particular application, the use of either whole blood or serum seems more suitable. This is highlighted on Figure 5.

The levels of IgG anti-TGase2 were measured in treated and untreated saliva samples from patients of all 5 groups. With the exception of a CD patient with IgAD from Group IV, no antibodies IgG anti-TGase2 was detected. These preliminary results are promising and seem to confirm the specificity of IgG anti-TGase2 in saliva to diagnose CD in patients with IgAD. However, more patient samples will be needed to positively conclude on the use of saliva to diagnose these patients.

Relating salivary immunoglobulins to systemic disease remains challenging. In the case of CD, two sub-classes of immunoglobulins have to be considered, IgA and IgG, each having a different origin in saliva. The former is synthetized by plasmocytes in salivary glands, and secreted in the form of dimers (secretory IgA). The latter is derived from serum mainly via gingival crevices and from oral mucosal transudate [6]. One of the challenges is to optimize saliva collection procedure in order to collect both IgA and IgG. By collecting saliva from under the tongue, IgA secreted from the sublingual gland was collected as well as IgG from the oral mucosal transudate. This has been previously demonstrated in the context of oral HIV testing [7]. It has the advantage of capturing both sIgA that reflect gut immunity from homing of activated B cells within minor salivary glands and IgG from serum.

While the invention has been illustrated by reference to specific and preferred embodiments, those skilled in the art will recognize that variations and modifications may be made through routine experimentation and practice of the invention. Thus, the invention is intended not to be limited by the foregoing description, but to be defined by the appended claims and their equivalents.

### REFERENCES

[1] Freitag T., Schulze-Koops H., Niedobitek G., et al. The role of immune response against tissue transglutaminase in the pathogenesis of celiac disease. Autoimmun. Rev. 2004; 3: 13-20.
[2] Esposito C. and Caputo I. Mammalian transglutaminases. Identification of substrates as a key to physiological function and pathophysiological relevance. FEBS. 2005; 272: 615-31.
[3] Molberg O., Solheim Flaete N., Jensen T., et al. Intestinal T-cell responses to high-molecular- weight glutenins in celiac disease. Gastroenterol. 2003; 125: 337-44.
[4] Lo W., Sano K., Lebwohl B., et al. Changing presentation of adult celiac disease. Dig. Dis. Sci. 2003; 48: 395-8.
[5] Lewis H.M., Renaula T.L., Garioch J.J., et al. Protective effect of gluten-free diet against development of lymphoma in dermatitis herpetiformis. Br. J. Dermatol. 1996; 135: 363-7.
[6] Tommasini A., Not T., Kiren V., et al. Mass screening for coeliac disease using antihuman transglutaminase antibody assay. Arch. Dis. Child. 2004; 89: 512-15.
[7] Mein S.M., Ladabaum U. Serological testing for celiac disease in patients with symptoms of irritable bowel syndrome: a cost-effective analysis. Aliment. Pharmacol. Ther. 2004; 19: 1199-1210.
[8] American Gastroenterological Association medical position statement: celiac sprue. Gastroenterol. 2001; 120: 1522-25.
[9] Robins G. and Howdle P.D. Advances in celiac disease. Curr. Opin. Gastroenterol. 2005; 21: 152-61.
[10] Fasano A. and Catassi C. Current approaches to diagnosis and treatment of celiac disease: an evolving spectrum. Gastroenterol. 2001; 120: 636-51.
[11] Sandolfi L., Pratesi R. Cordoba J.C., et al. Prevalence of celiac disease in healthy blood donors in Brazil. Am. J. Gastroenterol. 2000; 95: 689-92.
[12] Green P.H., and Jabri B. Coeliac disease. Lancet. 2003; 362: 383-91.
[13] Cataldo F., Marino V., Ventura A., et al. Prevalence and clinical features of selective immunoglobulin A deficiency in celiac disease: an Italian multicentre study. Italian Society of Paediatric Gastroenterology and Hepatology (SIGEP) and "Club del tenue" Working Group on Coeliac Disease. Gut. 1998; 42: 362-5.
[14] Cunningham-Rundles C. Physiology of IgA and IgA deficiency. J Clin. Immunol. 2001; 21: 303-9.
[15] Collin P., Maki M., Keyrilainen O., et al. Selective IgA deficiency and celiac disease. Scand. J. Gastroenterol. 1992; 27: 367-71.
[16] Revised criteria for diagnosis of celiac disease. Report of Working Group of European Society for Pediatric Gastroenterology and Nutrition. Arch. Dis. Child. 1990; 65: 909-11.
[17] Dieterich W, Ehnis T, Bauer M et al., Identification of Tissue Transglutaminase as the Autoantigen of Celiac Disease. Nat Med 197; 3: 797-801; WO 98/03872 Immumological Process for Detecting Antibodies Directed Towards Tissue Transglutaminase (TTG), Use of TTG for Diagnostic Purposes and Therapy Control, and Oral Pharmaceutical Agent Containing TTG, Schuppan D, Dieterich W.
[18] Salmaso C., Ocmant A., Pesce G., et al. Comparison of ELISA for tissue transglutaminase autoantibodies with antiendomysium antibodies in pediatric and adult patients with celiac disease. Allergy. 2001; 56(6): 544-7.
[19] Sorell L, Garrote JA, Acevedo B, et al., One-step Immunochromatographic Assay for Screening of Coeliac Disease. Lancet 2002; 359: 945-946. Patent EP 1,164,375, Assay for Anti-transglutaminase Antibodies, Sorell LT, Aroya H, Acevedo BE, Cerro H.
[20] Patent Application WO2004016065, Immunochromatography system and method for the simulataneous identification of AGA and T-TG antibodies and use thereof for the diagnosis of celiac disease, Mendez Corman E., Genzor Asin C.G., Gamen Sierra S., Corbaton Panplona V.M., Navarro Galindo A., Villacampa R.M.
[21] Guideline for the diagnosis and treatment of celiac disease in children: recommendations of the North American Society for Paediatric Gastroenterology, Hepatology and Nutrition. J. Pediatr. Gastroenterol. Nutr. 2005; 40: 1-19.
[22] Korponay-Szabo I.R., Kovacs J.B., Czinner A., et al. High prevalence of silent celiac disease in preschool children screened with IgA/IgG antiendomysium antibodies. J. Pediatr. Gastroenterol. Nutr. 1999; 28:26-30.
[23] Moher D. and Schachter H.M. Evidence report/technology assessment on celiac disease #104. AHRQ publication number 04-E029-2. 2004. www.ahrq.gov.
[24] Patent Application WO2005124344, Method for detecting anti-transglutaminase antibodies. Mascart F. and Ocmant C.
[25] Grebski E., Peterson C., and Medici T.C. Effect of physical and chemical methods of homogenization on inflammatory mediators in sputum of asthma patients. Chest. 2001; 119(5): 1521-5.
[26] Angeretti A., Merlino C., Ferrara B., et al. Effect of mercaptans on serum IgA. Boll. Ist. Sieroter. Milan. 1986 ; 65(5): 380-5.
[27] Patent CA1323832, Process for immunochromatography with colloidal particles, Shing S. and Gordon J.
[28] Single Step Heterogeneous Assay - (Immunochromatography) Inventor: Litman David Jay; Zuk Robert Frank; (+1) Applicant: SYNTEX INC EC: G01N33/535; G01N33/543K4 IPC: G01N33/535; G01N33/543; G01N33/535 (+2) Publication info: AU585505B - 1989-06-22
[29] Stazi A.V. and Trinti B. Reproduction, endocrine disorders and celiac disease: risk factors of osteoporosis. Minerva Med. 2006 Apr;97(2):191-203.
[30] Gass M. and Dawson-Hughes B. Preventing osteoporosis-related fractures: an overview. Am J Med. 2006 Apr;119(4 Suppl 1):S3-S11.
[31] Bonnick S.L. and Shulman L. Monitoring osteoporosis therapy: bone mineral density, bone turnover markers, or both? Am J Med. 2006 Apr;119(4 Suppl 1):S25-31.
[32] North American Menopause Society (NAMS). Management of osteoporosis in postmenopausal women: 2006 position statement of The North American Menopause Society. Menopause. 2006 May-Jun;13(3):340-67.
[33] Koka S., Forde M.D., and Khosla S. Systemic assessments utilizing saliva: part 2 osteoporosis and use of saliva to measure bone turnover. Int J Prosthodont. 2006 Jan-Feb;19(1):53-60.
[34] Jurjus Abdo R et al.; Diagnostics markers for Celiac disease in blood and body fluids. FASEB Journal, 1995, 19(5, suppl.. S, Part 2):pA 1068.
[35] Lahteenoja H et al.; Salivary antigliadin and antiendomysium antibodies in celiac disease. Scandinavian Journal of Immunology , 1999, 50/5, p528-535.

## Claims

1. An immunochromatography device for determining the presence of a binding analyte in a biological sample of a subject for simultaneously detecting the presence of total IgA and antibodies against TGase2 in at risk Celiac disease populations amongst which those with IgA deficiency, said binding analyte being an immunoglobulin selected from the group comprising an IgA, an sIgA, and IgG, or a combination thereof, comprising a sample application site for receiving said biological sample,
conjugate pads allowing said binding analyte present in said biological sample to bind to a labeled agent, and test pads comprising assay areas,
wherein
iii) a first conjugate pad is impregnated with a labeled anti human IgA and is in communication with a first test pad, said first test pad being coated, in the assay area, with tissue transglutaminase 2 (TGase2) or an antigenic part thereof and with anti human IgA or a binding portion thereof, and
iv) a second conjugate pad is impregnated with a labeled anti human IgG and is in communication with a second test pad, said second test pad being coated, in the assay area, with tissue transglutaminase 2 (TGase2) or an antigenic part thereof and with anti human IgG, a ubiquitous protein, or binding portions thereof,
and wherein the sample application site is adapted to direct at least part of said biological sample of a subject through said conjugate pads into at least three assay areas of said test pads and at least one of said test pads comprises two assay areas.

2. The immunochromatography device of claim 1, wherein the sample application site comprises at least one sample pad.

3. The immunochromatography device of any of claims 1 or 2, wherein one test pad is a control pad.

4. The immunochromatography device of any of claims 1-3, wherein the biological sample of a subject is selected from the group comprising whole blood, serum, plasma, saliva, urine, brain tissue, and cerebral spinal fluid.

5. The immunochromatography device of any of claims 1-4, wherein said immunochromatography device further comprises a recipient for a saliva collector device.

6. The immunochromatography device of any of claims 1- 5, wherein the sample application site comprises means for maintaining in contact the at least one sample pad with the biological sample of a subject.

7. The immunochromatography device of any of claims 1-6, wherein the sample application site is centered or de-centered.

8. The immunochromatography device of any of claims 1-7, further comprising soak pads.

9. A method for determining the presence of a binding analyte in a biological sample of a subject for simultaneously detecting the presence of total IgA and antibodies against TGase2 in at risk Celiac disease populations amongst which those with IgA deficiency comprising the steps of
a) providing the immunochromatography device of any of claims 1-8,
b) pre-treating said biological sample in a pre-treating buffer,
c) contacting the pre-treated biological sample of step b) with the sample pad of said immunochromatography device, thereby enabling said pre-treated biological sample to diffuse laterally into at least three assay areas of said test pads,
d) running a lateral flow assay thereby allowing the sample to flow through the sample pad toward the conjugate pads and then toward the test pads, wherein said binding analyte, being an immunoglobulin selected from the group comprising an IgA, an sIgA, and IgG, or a combination thereof, if present in said biological sample, first contacts either anti-human IgA or anti-human IgG so that complexes with said anti-human IgA or anti-human IgG are formed and wherein said complexes further contact with tissue transglutaminase 2 (TGase2) or an antigenic part thereof,
e) enabling the development of a response and detecting the presence of the label present on the complexes,
f) interpreting the response to indicate the presence of said binding analyte in said biological sample.

10. The method of claim 9, wherein the pre-treating buffer is selected from the group comprising N-acetyl-cystein in PBS buffer or carbocystein in PBS buffer.

11. The method of any of claims 9-10, wherein the label is selected from the group comprising chromogens, catalysts, fluorescent compounds, colloidal metallic and nonmetallic particles, dye particles, enzymes or substrates, organic polymers, latex particles, and liposomes with signal producing substances.

12. A kit for determining the presence of a binding analyte in a biological sample of a subject, said kit comprising the immunochromatography device of claims 1-8, optionally with reagents and/or instructions for use.

13. The kit of claim 12, further comprising a saliva collector device.

## Patentansprüche

1. Eine immunchromatographische Vorrichtung zur Bestimmung des Vorhandenseins eines bindenden Analyten in einer biologischen Probe eines Subjekts zur gleichzeitigen Ermittlung des Vorhandenseins des Gesamt-IgA und von Antikörpern gegen TGase2 in Zöliakie-Risikogruppen inklusive diejenigen mit IaA-Mangel, wobei der bindende Analyt ein Immunglobulin ist, ausgewählt aus der Gruppe enthaltend ein IgA, ein sIgA und IgG oder einer Kombination davon, mit
einer Pobenanwendungsstelle zur Aufnahme der besagten biologischen Probe, Konjugat-Pads die es erlauben, den besagten, in besagter biologischer Probe vorhandenen, bindenden Analyten an ein Markiermittel zu binden,
und Testpads, welche Assaybereiche enthalten, worin
iii) ein erstes Konjugat-Pad mit einem markierten Anti-human-IgA imprägniert wird und in Verbindung mit einem ersten Testpad ist, wobei das besagte erste Testpad im Assaybereich mit Gewebetransglutaminase 2 (TGase2) oder einem antigenen Teils davon und mit Anti-human-IgA oder einem bindenden Teil davon überzogen wird, und
iv) ein zweites Konjugat-Pad mit einem markierten Anti-human-IgG imprägniert wird und in Verbindung mit einem zweiten Testpad ist, wobei das besagte zweite Testpad im Assaybereich mit Gewebetransglutaminase 2 (TGase2) oder einem antigenen Teil davon und mit Anti-human-IgG, einem allgegenwärtigen Protein, oder bindenden Teilen davon überzogen wird,
und worin die Pobenanwendungsstelle angepasst ist, um wenigstens Teile der besagten biologischen Probe eines Subjekts durch die besagten Konjugat-Pads in mindestens drei Assaybereiche der besagten Testpads zu leiten und worin mindestens eines der besagten Testpads zwei Assaybereiche umfasst.

2. Die immunchromatographische Vorrichtung von Anspruch 1, worin die Pobenanwendungsstelle mindestens ein Probenpad enthält.

3. Die immunchromatographische Vorrichtung irgendeines der Ansprüche 1 oder 2, worin ein Testpad ein Kontrollpad ist.

4. Die immunchromatographische Vorrichtung irgendeines der Ansprüche 1 - 3, worin die biologische Probe eines Subjekts ausgewählt wird aus der Gruppe enthaltend Vollblut, Serum, Plasma, Speichel, Urin, Hirngewebe und Rückenmarksflüssigkeit.

5. Die immunchromatographische Vorrichtung irgendeines der Ansprüche 1 - 4, worin besagte immunchromatographische Vorrichtung zudem einen Empfänger für eine Speichelsammlungsvorrichtung aufweist.

6. Die immunchromatographische Vorrichtung irgendeines der Ansprüche 1 - 5, worin die Pobenanwendungsstelle Mittel enthält, um das mindestens eine Probenpad mit der biologischen Probe eines Subjekts in Kontakt zu halten.

7. Die immunchromatographische Vorrichtung irgendeines der Ansprüche 1 - 6, worin die Pobenanwendungsstelle zentriert oder de-zentriert ist.

8. Die immunchromatographische Vorrichtung irgendeines der Ansprüche 1 - 7, zudem mit Einweichpads.

9. Eine Verfahren zur Bestimmung des Vorhandenseins eines bindenden Analyten in einer biologischen Probe eines Subjekts zur gleichzeitigen Ermittlung des Vorhandenseins aller IgA und von Antikörpern gegen TGase2 in Zöliakie-Risikogruppen inklusive diejenigen mit IaA-Mangel, mit den folgenden Schritten:
a) Zurverfügungstellung der immunchromatographischen Vorrichtung irgendeines der Ansprüche 1 - 8,
b) Vorbehandlung der besagten biologischen Probe in einem Vorbehandlungs-Puffer,
c) Kontaktierung der vorbehandelten Probe vom Schritt b) mit dem Probenpad der besagten immunchromatographischen Vorrichtung, was der besagten vorbehandelten Probe ermöglicht, sich lateral in mindestens drei Assaybereiche der besagten Testpads auszubreiten,
d) Ausführung eines Lateral Flow Assays, was der Probe ermöglicht, durch das Probenpad gegen die Konjugate-Pads und dann gegen die Testpads zu fliessen, worin der besagte bindende Analyt, als Immunglobulin ausgewählt aus der Gruppe enthaltend ein IgA, ein sIgA und IgG oder einer Kombination davon, falls in besagter biologischer Probe vorhanden, zuerst entweder Anti-human-IgA oder Anti-human-IgG kontaktiert, so dass Komplexe mit besagtem Anti-human-IgA oder Anti-human-IgG geformt werden, und worin die besagten Komplexe zudem Gewebetransglutaminase 2 (TGase2) oder einen antigenen Teil davon kontaktieren,
e) die Entwicklung einer Reaktion und die Ermittlung des Vorhandenseins der auf den Komplexen vorhandenen Markierung ermöglichen,
f) Interpretieren der Reaktion, um das Vorhandensein des besagten bindenden Analyten in besagter biologischer Probe anzuzeigen.

10. Das Verfahren von Anspruch 9, worin der Vorbehandlungs-Puffer ausgewählt wird aus der Gruppe mit N-Acetyl-Cystein im PBS-Puffer oder Carbocystein im PBS-Puffer.

11. Das Verfahren irgendeines der Ansprüche 9 -10, worin die Markierung ausgewählt wird aus der Gruppe enthaltend Chromogene, Katalysatoren, fluoreszierende Verbindungen, metallische und nicht-metallische Kolloidteilchen, Farbstoffpartikel, -enzyme oder -substrate, organische Polymere, Latex-Partikel und Liposome mit anzeigenproduzierenden Substanzen.

12. Ein Kit zur Bestimmung des Vorhandenseins eines bindenden Analyten in einer biologischen Probe eines Subjekts, wobei das besagte Kit die besagte immunchromatographische Vorrichtung der Ansprüche 1 - 8 aufweist, wahlweise mit Reagenzien und/oder Gebrauchsanweisung.

13. Das Kit von Anspruch 12, zudem mit einer Speichelsammlungsvorrichtung.

## Revendications

1. Dispositif d'immunochromatographie pour déterminer la présence d'un analyte liant dans un échantillon biologique provenant d'un sujet pour simultanément détecter la présence de quantité totale d'IgA et d'anticorps contre TGase2 dans les populations à risque de maladie coeliaque y compris présentant une carence en IgA, ledit analyte liant étant une immunoglobuline sélectionnée parmi le groupe comprenant un IgA, un sIgA et IgG, ou leur combinaison, comprenant
un site d'application d'échantillon pour recevoir ledit échantillon biologique,
des supports conjugués permettant audit analyte liant présent au sein dudit échantillon biologique pour se lier à un agent marqué, lesdits supports de test comprenant des zones d'analyse,
dans lequel
iii) le premier support est imprégné avec un IgA marqué antihumain et est en communication avec un premier support de test, ledit premier support de test étant revêtu, dans la zone d'analyse, de transglutaminase 2 tissulaire (TGase2) ou d'un élément antigénique de celle-ci, et d'IgA antihumain ou d'une portion liante de celle-ci, et
iv) le deuxième support est imprégné avec un IgG marqué antihumain et est en communication avec un deuxième support de test, ledit deuxième support de test étant revêtu, dans la zone d'analyse, de transglutaminase 2 tissulaire (TGase2) ou d'un élément antigénique de celle-ci, et d'IgA antihumain, de protéine omniprésente ou de portions liantes de celle-ci,
et dans lequel
le site d'application d'échantillon est adapté pour mener au moins une partie dudit échantillon biologique provenant d'un sujet au travers desdits supports conjugués dans au moins trois zones d'analyse desdits supports de test et au moins un desdits supports de test comprend deux zones d'analyse.

2. Le dispositif d'immunochromatographie de la revendication 1, dans lequel le site d'application d'échantillon comprend au moins un support d'échantillonnage.

3. Le dispositif d'immunochromatographie de l'une quelconque des revendications 1 ou 2, dans lequel un support de test est un support de contrôle.

4. Le dispositif d'immunochromatographie de l'une quelconque des revendications 1 à 3, dans lequel l'échantillon biologique provenant d'un sujet est choisi parmi le groupe comprenant: sang entier, sérum, plasma, salive, urine, tissu cérébral et liquide céphalo-rachidien.

5. Le dispositif d'immunochromatographie de l'une quelconque des revendications 1 à 4, dans lequel ledit dispositif d'immunochromatographie comprend en outre un récipient pour dispositif de prélèvement de salive.

6. Le dispositif d'immunochromatographie de l'une quelconque des revendications 1 à 5, dans lequel le site d'application d'échantillon comprend des moyens pour maintenir en contact le au moins un support d'échantillonnage avec l'échantillon biologique provenant d'un sujet.

7. Le dispositif d'immunochromatographie de l'une quelconque des revendications 1 à 6, dans lequel le site d'application d'échantillon est centré ou décentré.

8. Le dispositif d'immunochromatographie de l'une quelconque des revendications 1 à 7, comprenant en outre des supports de trempage.

9. Procédé pour déterminer la présence d'un analyte liant dans un échantillon biologique provenant d'un sujet pour simultanément détecter la présence de quantité totale d'IgA et d'anticorps contre TGase2 dans les populations à risque de maladie coeliaque y compris présentant une carence en IgA, comprenant les étapes de :
a) mettre à disposition le dispositif d'immunochromatographie de l'une quelconque des revendications 1 à 8,
b) prétraiter ledit échantillon biologique dans un tampon de prétraitement,
c) contacter l'échantillon biologique prétraité de l'étape b) avec le support d'échantillonnage dudit dispositif d'immunochromatographie, permettant ainsi audit échantillon biologique prétraité de diffuser latéralement dans au moins trois zones d'analyse desdits supports de test,
d) exécuter une analyse à écoulement latéral permettant ainsi à l'échantillon de circuler à travers le support d'échantillonnage vers les supports conjugués et ensuite vers les supports de test, dans lequel ledit analyte liant, étant une immunoglobuline sélectionnée parmi le groupe comprenant un IgA, un sIgA, et IgG ou leur combinaison, lorsqu'il est présent au sein dudit échantillon biologique, contacte premièrement soit l'IgA antihumain ou l'IgG antihumain en sorte que des complexes avec ledit IgA antihumain ou IgG antihumain sont formés, et dans lequel lesdits complexes contactent en outre la transglutaminase 2 tissulaire (TGase2) ou une partie antigénique de celle-ci,
e) permettre le développement d'une réponse et détecter la présence du marquage présent sur les complexes,
f) interpréter la réponse pour indiquer la présence dudit analyte liant dans ledit échantillon biologique.

10. Le procédé de la revendication 9, dans lequel le tampon de prétraitement est sélectionné parmi le groupe comprenant N-acétylcystéine dans un tampon phosphate-salin PBS ou carbocystéine dans un tampon PBS.

11. Le procédé selon l'une quelconque des revendications 9-10, dans lequel le marquage est sélectionné parmi le groupe comprenant les chromogènes, catalyseurs, composés fluorescents, particules colloïdales métalliques et non-métalliques, particules ou enzymes ou substrats colorants, polymères organiques, particules de latex et liposomes avec substances produisant un signal.

12. Un kit déterminant la présence d'un analyte liant dans un échantillon biologique provenant d'un sujet, ledit kit comprenant le dispositif d'immunochromatographie des revendications 1 à 8, avec en option des réactifs et/ou instructions d'utilisation.

13. Le kit de la revendication 12, comprenant en outre un dispositif de prélèvement de salive.
